# EUROPEAN PATENT APPLICATION

(11) **EP 1 192 909 A1**
(43) Date of publication of application: **03.04.2002**
(21) Application number: 00121017.8
(22) Date of filing: 27.09.2000
(51) Int. Cl.: A61B 17/68

(54) **Post-surgical fixation device for skull**

(71) Applicant: Yeh, Chung-Chun, Taipei (TW)
(72) Inventor: Yeh, Chung-Chun, Taipei (TW)
(74) Representative: Grünecker, Kinkeldey, Stockmair & Schwanhäusser Anwaltssozietät

(57) **Abstract**

A skull post-surgical fixation device includes an adjustment pull bar (100), a lower fixation piece (200), and an upper fixation piece (300). The lower fixation piece is connected with the bottom end of the adjustment pull bar. The adjustment pull bar is provided near the bottom end thereof with a first uniting part (120). The upper fixation piece has a through hole (310) for receiving the adjustment pull bar. The through hole is provided with a second uniting part (320) cooperating with the first uniting part of the adjustment pull bar. The first uniting part of the adjustment pull bar and the second uniting part of the upper fixation piece form an adjustable and slidable uniting mechanism.

## Description

### FIELD OF THE INVENTION

The present invention relates to a post-surgical fixation device for skull.

### BACKGROUND OF THE INVENTION

The conventional post-surgical fixation device for skull generally utilizes a wire or a craniotomy pin to hold the separated skull. For example, U. S. Pat. No. 5,549,620 discloses a skull fixation device which is formed of an upper plate, a lower plate, and a clamp rod. The skull must be drilled a round hole to facilitate the inserting of the skull fixation device. The drilling of the round hole in the skull prolongs the surgery as well as the healing of the skull.

It is the primary objective of the present invention to provide a post-surgical fixation device for skull.

It is another objective of the present invention to provide a post-surgical fixation device for skull, which has an adjustable sliding uniting mechanism.

It is still another objective of the present invention to provide a post-surgical fixation device for skull, which has an adjustable sliding uniting mechanism, and is formed of an adjustment pull bar, a lower fixation piece, and an upper fixation piece. The uniting mechanism is easy to be separated when the upper fixation piece is pushed to advance along the adjustment pull bar, and is difficult to be separated when the upper fixation piece is pull to retreat from the adjustment pull bar.

### SUMMARY OF THE INVENTION

In order to accomplish the objectives of the present invention a skull post-surgical fixation device constructed according to the present invention comprises:
an adjustment pull bar provided near a bottom end thereof with a first uniting means;
a lower fixation piece connected with the bottom end of said adjustment pull bar; and
an upper fixation piece having a through hole for receiving said adjustment pull bar, said through hole provided with a second uniting means cooperating with said first uniting means of said adjustment pull bar;
wherein said first uniting means and said second uniting means form an adjustable and slidable uniting mechanism, so that the first and second uniting means will be detached from each other when said upper fixation piece is pushed to advance along said adjustment pull bar, and the first and second uniting means will be locked when said upper fixation piece is pull to retreat from said adjustment pull bar.

Preferably, said lower fixation piece is fixedly connected with the bottom end of said adjustment pull bar, so that said lower fixation piece and said adjustment pull bar become integral.

Preferably, said adjustment pull bar is substantially cylindrical, rectangular, or square bar.

Preferably, said first uniting means of said adjustment pull bar comprises parallel slots; wherein said second uniting means of said upper fixation piece comprises ratcheting teeth.

Alternatively, said first uniting means of said adjustment pull bar comprises ratcheting teeth; wherein said second uniting means of said upper fixation piece comprises parallel slots.

### BRIEF DESCRIPTION OF THE DRAWINGS

FIG. 1 shows a top perspective view of the upper fixation piece of the post-surgical fixation device for skull of the present invention.
FIG. 2 shows a bottom perspective view of the upper fixation piece of the skull post-surgical fixation device of the present invention.
FIG. 3 shows a top plan view of the upper fixation piece of the skull post-surgical fixation device of the present invention.
FIG. 4 shows a cross sectional view of the upper fixation piece of the skull post-surgical fixation device of the present invention.
FIG. 5 shows a perspective view of the adjustment pull bar and the lower fixation piece of the skull post-surgical fixation device of the present invention.
FIG. 6A and 6B are schematic views showing the way by which the skull post-surgical fixation device of the present invention is united.
FIG 7 is a schematic view showing the completion of the union of the skull post-surgical fixation device of the present invention after the removal of the excess portion of the adjustment pull bar thereof.
FIGS. 8A-8D are schematic views showing the skull post-surgical fixation device of the present invention in operation.
FIG. 9 shows a schematic view of the present invention at the conclusion of the operation as shown in FIGS. 8A-8D and after the removal of the excess portion of the adjustment pull bar thereof.
FIGS. 10A and 10B are schematic views showing the process of assembling of a post-surgical fixation device for skull according to another preferred embodiment of the present invention in combination.

### DETAILED DESCRIPTION OF THE EMBODIMENTS

The skull post-surgical fixation device of the present invention comprises an adjustment pull bar, a lower fixation piece, and an upper fixation piece.

The lower fixation piece is connected with a bottom end of the adjustment pull rod. The adjustment pull bar is provided near the bottom end thereof with a first uniting means. The upper fixation piece has a through hole and a second uniting means on a side wall or side walls of the through hole. The upper fixation piece is united with the adjustment pull bar such that the adjustment pull bar is received in the through hole of the upper fixation piece. The second uniting means of the upper fixation piece cooperates with the first uniting means of the adjustment pull bar.

The present invention is characterized in that said first uniting means and said second uniting means form an adjustable and slidable uniting mechanism, so that the first and second uniting means will be detached from each other when said upper fixation piece is pushed to advance along said adjustment pull bar, and the first and second uniting means will be locked when said upper fixation piece is pull to retreat from said adjustment pull bar.

The adjustment pull bar of the present invention has a length, which is preferably in the range of 3 and 10cm. Upon removal of the excess portion, the adjustment pull bar will have a length which is about equal to the sum of the thickness of the skull and the thickness of the upper fixation piece.

The adjustment pull bar has a pull bar portion which is of any shape and is preferably columnar, cylindrical, rectangular columnar, or square columnar. The shape referred to above is the cross-sectional shape. The shape may be slightly changed to an extent that the corners of the rectangular or square column may be of an arcuate shape.

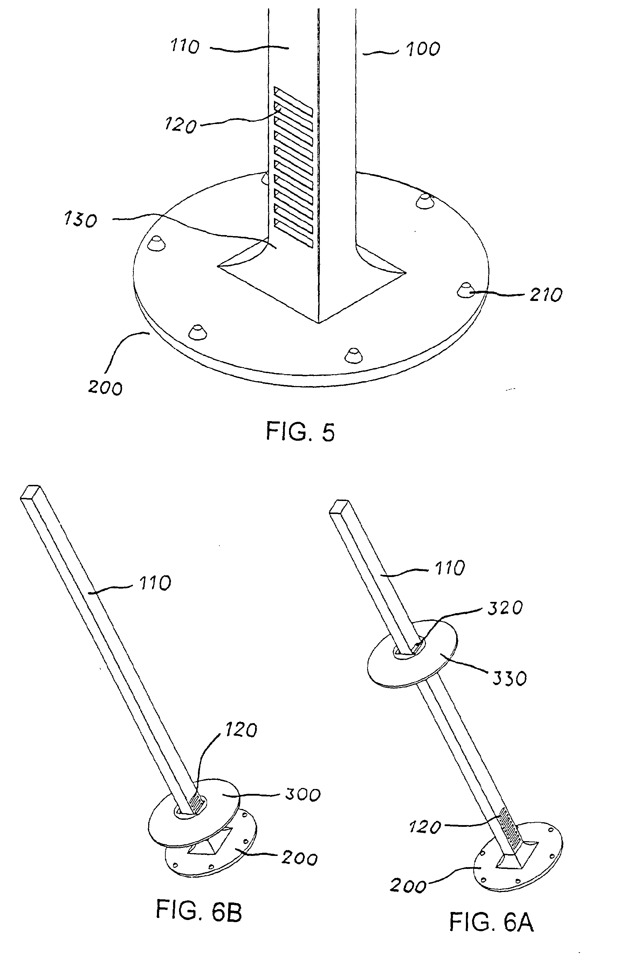

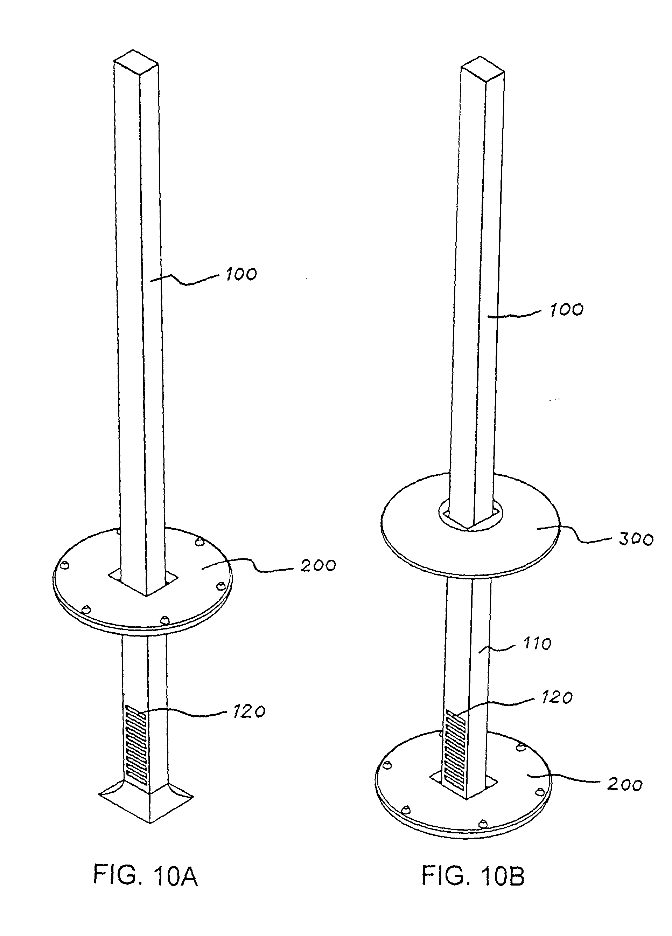

The lower fixation piece may be connected with the bottom end of the adjustment pull bar in a way such that they are integrally formed, as shown in FIG. 5, or that they are made separately and then joined together by the conventional method, such as the hook-and-eye uniting mechanism or the mortise-tenon uniting mechanism. For example, the lower fixation piece is a fixation piece having a uniting means capable of uniting with the second uniting means of the adjustment pull bar. The way that the separate bodies are united together may be a simple fitting method, as shown in FIG. 10A, wherein the lower fixation piece may be a washer directly received at an expanded bottom end of the adjustment pull bar.

The through hole of the upper fixation piece may be located at any portion of the upper fixation piece, preferably in the center of the upper fixation piece. The through hole is of a shape corresponding to the profile of the pull bar portion of the adjustment pull bar. If the pull bar portion is rectangular in shape, the through hole should be also rectangular in shape.

The second uniting means of the through hole cooperates with the first uniting means provided at least near the bottom end of the adjustment pull bar. The first uniting means and the second uniting means form an adjustable and slidable uniting mechanism which may be a conventional adjustable and slidable uniting mechanism, such as the hook-and-eye uniting mechanism, the mortise-tenon uniting mechanism. For example, if the first uniting means of the adjustment pull bar contains parallel slots, the second uniting means of the through hole should be ratcheting teeth engageable with the slots, or vice versa. If the adjustment pull bar is of a rectangular columnar shape or square columnar shape, the first uniting means thereof is preferably located at two opposite planar surfaces. Accordingly, the second uniting means of the through hole is located at two opposite side walls.

The lower fixation piece has a shape capable of cooperating with the convex shape of the skull, or the planar shape or the concave shape. The convex shape and the concave shape are slightly curved.

The upper fixation piece has a convex shape, the planar shape or the concave shape, and preferably, the convex shape. The convex shape and the concave shape are slightly curved.

The word "lower" or "bottom end" refers to the direction of a standing patient toward the ground. The opposite direction of "lower" is "upper". The opposite direction of "bottom end" is "top portion".

If necessary, the upper fixation piece and/or the lower fixation piece may be provided with a reinforcing structure, such as a reinforcing ring and/or a reinforcing rib. Other structures may be provided such as the structure for enhancing the union of the fixation piece with the skull. For example, the lower fixation piece is provided at a contact portion with the skull with protrusions or rugged surface, such as the rugged strips, for enhancing the union of the lower fixation piece with the skull.

The skull post-surgical fixation device of the present invention is made of an orthopedic biomaterial which is biologically compatible.

The present invention is further described hereinafter by a preferred embodiment with reference to the accompanying drawings.

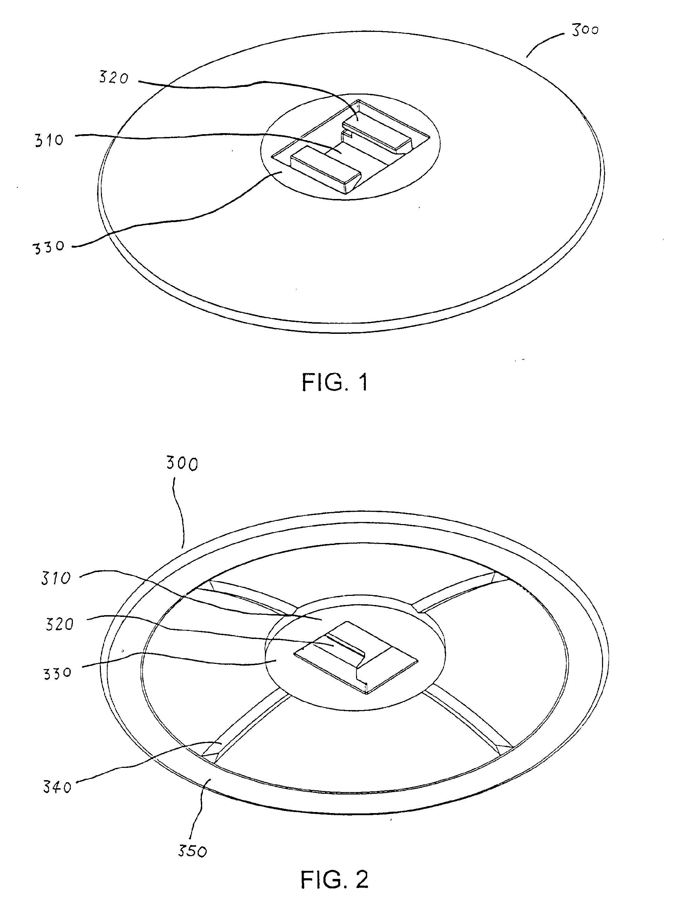

As shown in FIGs. 1 to 4, an upper fixation piece 300 of the skull post-surgical fixation device according to a first preferred embodiment of the present invention is provided with a through hole 310, teeth 320, a reinforcing ring 330. The upper fixation piece 300 has a convex shape. The lower concave surface of the upper fixation piece 300 is provided with four reinforcing ribs 340, and an outer annular contact surface 350.

As shown in FIG. 5, an adjustment pull bar 100 is provided with a pull bar portion 110, parallel slots 120, a bottom end 130. A lower fixation piece 200 is provided with protrusions 210 and is integrally made with the adjustment pull bar 100.

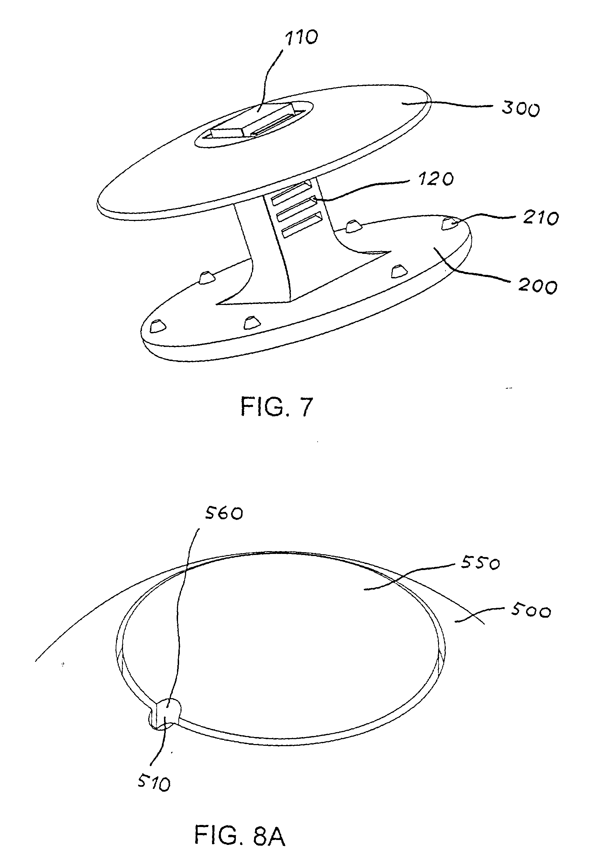

The reference numerals of FIGs. 6A, 6B, and 7 are similar in definition to those of the above Figures.

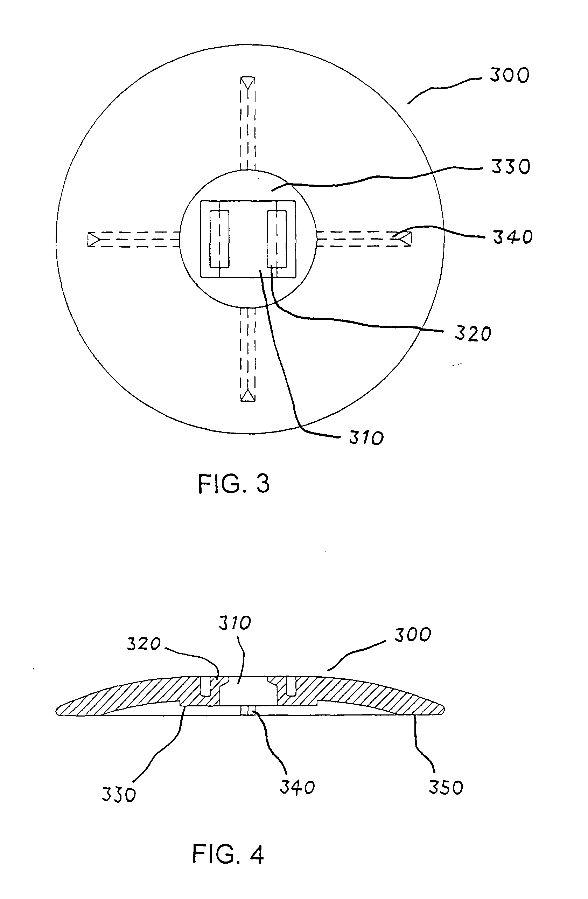

As shown in FIG. 6A, the through hole of the upper fixation piece 300 is engaged with the pull bar portion 110 of the adjustment pull bar 100. When the teeth 320 of the upper fixation piece 300 is engaged with the parallel slots 120 of the adjustment pull bar, the bevels of the teeth 320 (as shown in FIGs. 1 and 4) cooperates with the bevels of the parallel slots 120 (as shown in FIG. 5), thereby forming "easy advance" uniting mechanism (easy advance of the upper fixation piece 300 along the adjustment pull bar 100). The upper planar surfaces of the teeth 320 are substantially perpendicular to the advance-retreat direction of the upper fixation piece 300, as well as the upper planar surface of the parallel slots 120, and thus result in the "difficult retreat" uniting mechanism.

As shown in FIG. 7, the excess portion of the pull bar portion 110 is removed upon completion of fixation.

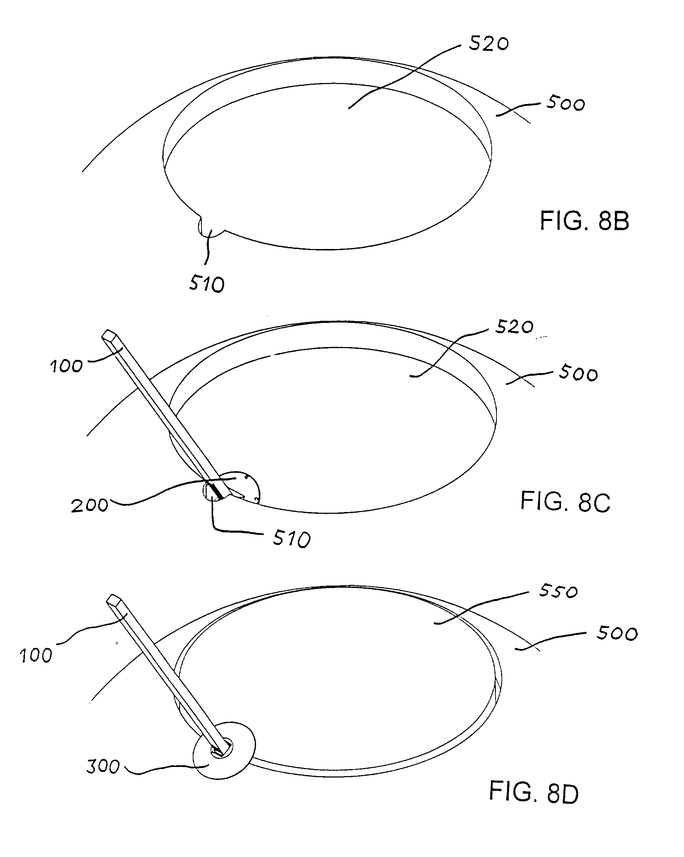

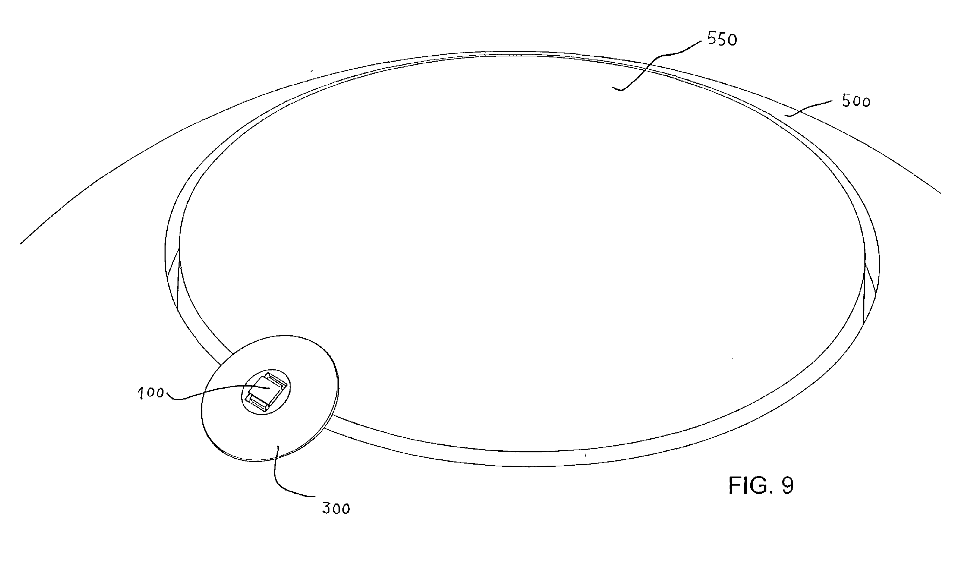

As shown in FIGs. 8A-8D, a temporarily-removed skull 550 is fixed to the operated skull 500 having two incisions 510 and 560 by using the skull post-surgical fixation device according to the first preferred embodiment of the present invention of the present invention. FIG. 8A shows the temporarily-removed skull 550 before being removed from the operated skull 500. FIG. 8B shows the temporarily-removed skull 550 after being removed. After being removed, the operated skull 500 is provided with an incision 520. As shown in FIG. 8C, the adjustment pull bar 100 is disposed in the incision 510 after the lower fixation piece 200 is entered into the incision 520. As shown in FIG. 8D, the temporarily-removed skull 550 is returned to the original position. The upper fixation piece 300 is fitted, pushed, and located. Finally, the excess portion of the pull bar portion 110 is removed, as shown in FIG. 9. The reference numerals of FIG. 9 are similar in definition to those of the above Figures.

Generally speaking, the skull post-surgical fixation may be provided with three fixation devices. In FIGs. 8A, 8B, 8C, 8D, and 9 only one of them is used. Generally speaking, as shown in FIG. 8A, the skull 500 and the incised skull 550 are provided therebetween with an incision, which is sufficient to accommodate the adjustment pull bar 100 of the present invention. As a result, even without the incisions 510 and 560, the skull post-surgical fixation device of the present invention can be disposed.

The reference numerals of FIGs. 10A, 10B are similar in definition to those of FIGs. 6A and 6B. In FIGs. 10A and 10B, the adjustment pull bar 100 and the lower fixation piece 200 are not made integrally and are fitted together. FIG. 10A shows the fitting form of the adjustment pull bar 100 and the lower fixation piece 200, wherein the lower fixation piece 200 similar to a washer is received at an expanded bottom end of the adjustment pull bar 100. FIG. 10B shows that the upper fixation piece 300 is fitted with the pull bar portion 110. The adjustment pull bar 100 and the upper fixation piece 300 are united by the same way as described above.

## Claims

1. A skull post-surgical fixation device comprising:
an adjustment pull bar provided near a bottom end thereof with a first uniting means;
a lower fixation piece connected with the bottom end of said adjustment pull bar; and
an upper fixation piece having a through hole for receiving said adjustment pull bar, said through hole provided with a second uniting means cooperating with said first uniting means of said adjustment pull bar;
wherein said first uniting means and said second uniting means form an adjustable and slidable uniting mechanism, so that the first and second uniting means will be detached from each other when said upper fixation piece is pushed to advance along said adjustment pull bar, and the first and second uniting means will be locked when said upper fixation piece is pull to retreat from said adjustment pull bar.

2. The device as defined in claim 1, wherein said lower fixation piece is fixedly connected with the bottom end of said adjustment pull bar, so that said lower fixation piece and said adjustment pull bar become integral.

3. The device as defined in claim 1, wherein said adjustment pull bar is substantially cylindrical, rectangular, or square bar.

4. The device as defined in claim 1, wherein said first uniting means of said adjustment pull bar comprises parallel slots; wherein said second uniting means of said upper fixation piece comprises ratcheting teeth.

5. The device as defined in claim 1, wherein said first uniting means of said adjustment pull bar comprises ratcheting teeth; wherein said second uniting means of said upper fixation piece comprises parallel slots.
